# EUROPEAN PATENT APPLICATION

(11) **EP 4 052 732 A1**
(43) Date of publication of application: **07.09.2022**
(21) Application number: 21160636.3
(22) Date of filing: 04.03.2021
(51) Int. Cl.: A61L 2/10, B66B 11/00, F21V 21/00, H01H 13/00

(54) **A STERILISATION SYSTEM**

(71) Applicant: Uvsens Limited, D04 ED73 Dublin (IE)
(72) Inventor: DEITHRICH, Leon, Bray, Wicklow A98 Y7K5 (IE)
(74) Representative: White, Jonathan Patrick

(57) **Abstract**

The present invention relates to a sterilisation system for sterilising a surface comprising ultraviolet (UV) light emitting means operable to emit UV-C light radiation, a mounting for the ultraviolet (UV) light emitting means, moving means operable to move the mounting, sensor means operable to detect when the surface is touched by a human, control means operable to activate the moving means and the ultraviolet (UV) light emitting means in response to signals from the sensor means, whereby when the sensor means detects that the surface has been touched the control means is operable to activate the moving means to move the mounting and simultaneously and automatically activate the UV light emitting means such that UV-C light radiation emitted from the UV light emitting means is directed onto the surface to sterilise the surface. The present invention may be adapted for use in sterilising surfaces of elevator interfaces, access control panels for exit and entry to buildings, user terminal for operating a credit/debit card transaction, ATM interfaces and the like.

## Description

The present invention relates to a sterilisation system that utilises ultraviolet (UV) light to kill bacteria, germs and viruses on surfaces, such as, but not limited to, elevator control panels, credit card reading machines and other keypad operated devices.

The transfer of germs, bacteria and viruses is well known to lead to the spread and development of infections and illnesses. Transfer of such contaminants is prevalent in public spaces where individuals are forced to touch and operate many different types of keypads, touch screens and elevators buttons.

The current practice of using cleaning agents and chemicals on a regular basis helps to protect the public to a certain extent but due to ever growing populations and crowded urban areas this practice cannot keep up with the sheer volume of human traffic using these devices all day every day. The cost of these cleaning agents and hiring of staff to maintain these public points of contamination is proving costly to the economy and unable to keep these areas clean regularly enough. Moreover, these cleaning agents are not only expensive but also toxic to manufacture and use.

It is a therefore an object of the present invention to provide a sterilisation system which goes at least some way toward overcoming the above problems and/or which will provide the public and/or industry with a useful alternative.

Further aspects of the present invention will become apparent from the ensuing description which is given by way of example only.

According to the invention, there is provided a sterilisation system for sterilising a surface comprising:
ultraviolet (UV) light emitting means operable to emit UV-C light radiation,
a mounting for the ultraviolet (UV) light emitting means,
moving means operable to move the mounting,
sensor means operable to detect when the surface is touched by a human,
control means operable to activate the moving means and the ultraviolet (UV) light emitting means in response to signals from the sensor means,
whereby when the sensor means detects that the surface has been touched the control means is operable to activate the moving means to move the mounting and simultaneously and automatically activate the UV light emitting means such that UV-C light radiation emitted from the UV light emitting means is directed onto the surface to sterilise the surface.

Preferably, control means activates the moving means and the UV light emitting means in timed activation cycles.

Preferably, the sensor means is operable to detect placement and removal of a human user's hand on the surface.

Preferably, the mounting comprises a mounting bar and guide means, and the bar moves relative to the guide means to direct UV light onto the surface.

Preferably the guide means is provided as a pair of spaced apart slide rails provided at or adjacent edges of the surface, and the mounting bar moves along the slide rails over the surface. The guide rails and mounting bar will be modifiable to fit different size user interfaces.

Preferably, the moving means comprises one or more motors operable to move the mounting bar along the slide rails. The mounting bar may move up and down the slide rails, or from side to side along the slide rails depending on the orientation of the rails to the surface being sterilised.

It will be understood that the mounting bar and guide means are configured to move the UV light emitting means over the surface such that the UV light is directed onto the entire area thereof to thereby sterilise the full exposed area of the surface by the action of UV light emitted by the UV light emitting means.

The motors may be DC motors, in which a motor is provided at each guide rail.

Preferably, the ultraviolet (UV) light emitting means comprises one or more LED lights configured to emit UV-C light radiation. The LED lights may be provided as a strip of LED lights on the mounting bar.

In the following, the term sterilising wavelength thus refers to a light wavelength that can kill, neutralize or otherwise remove contaminant. The sterilising wavelength is in the range of 240nm to 270nm (nanometres) in the C spectrum with 247nm being most preferred.

Preferably, the ultraviolet (UV) light emitting means is configured to emit UV-C light at approximate waves length of between 240nm to 270nm.

Preferably, the ultraviolet (UV) light emitting means is configured to emit UV-C light at approximate waves length of between 250nm to 260nm.

Preferably, the ultraviolet (UV) light emitting means is configured to emit UV-C light at approximate waves length of approximately 250nm and more optimally 254nm.

Preferably, the sensor is configured to detect motion within 5 inches of the surface.

Preferably, a user interface is provided on the surface and is sterilised by the ultraviolet (UV) light emitting means.

The user interface may be an elevator control panel with buttons corresponding to floor levels of a building and functions of the elevator, a keypad or user terminal for operating a credit/debit card transaction, an ATM interface, or the like.

Preferably, the motion sensor is further configured to detect proximity of a user's hand to the surface and the ultraviolet (UV) light emitting means cycle will deactivate when a user's hand is detected as being within a predetermined distance of the surface. In this instance the mounting bar will then return to a starting position until such time as there is no further movement detected by the sensor means and will restart the sterilisation cycle again.

According to the invention, there is also provided a sterilisation system for sterilising user interface surfaces of an elevator system comprising:
ultraviolet (UV) light emitting means operable to emit UV-C light radiation,
a mounting for the ultraviolet (UV) light emitting means provided adjacent the interior button panel of the elevator,
moving means operable to move the mounting,
sensor means operable to detect when the surface is touched by a human,
control means operable to activate the moving means and the ultraviolet (UV) light emitting means in response to signals from the sensor means,
whereby when the sensor means detects that the surface has been touched the control means is operable to activate the moving means to move the mounting and simultaneously and automatically activate the UV light emitting means such that UV-C light radiation emitted from the UV light emitting means is directed onto the surface to sterilise the surface.

Preferably, the sterilisation system further comprises a secondary sterilisation unit comprising a further UV light emitting means and sensor means, in which the secondary sterilisation unit is provided adjacent the exterior call button for the elevator.

A light shield is provided with the secondary sterilisation unit to prevent a user being exposed to the emitted UV-C light radiation.

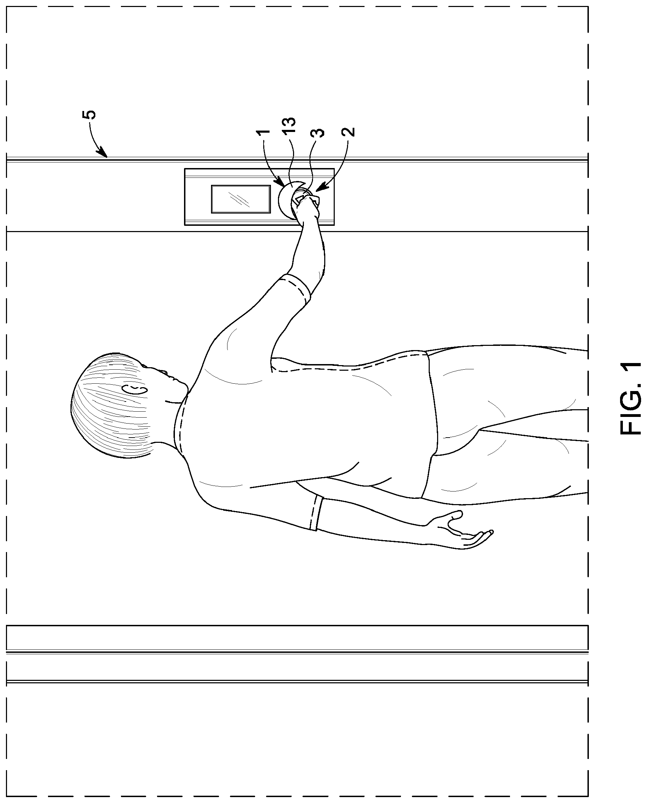

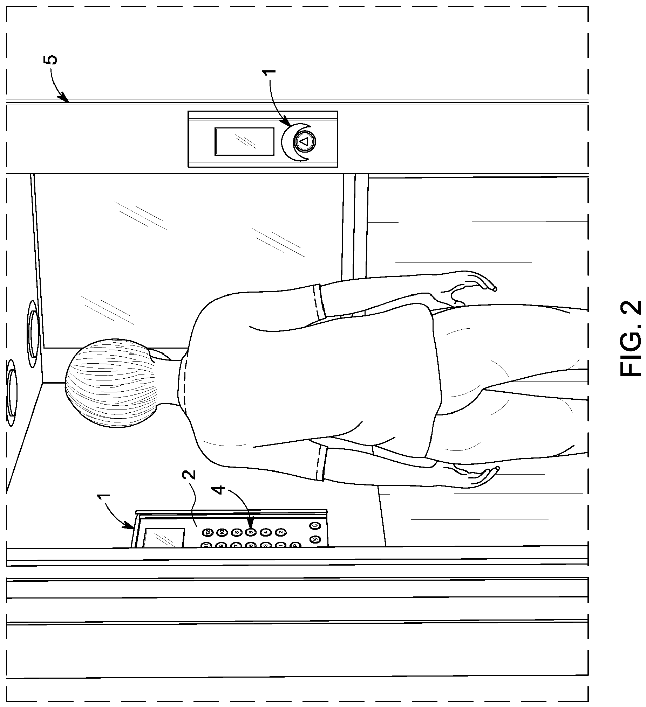

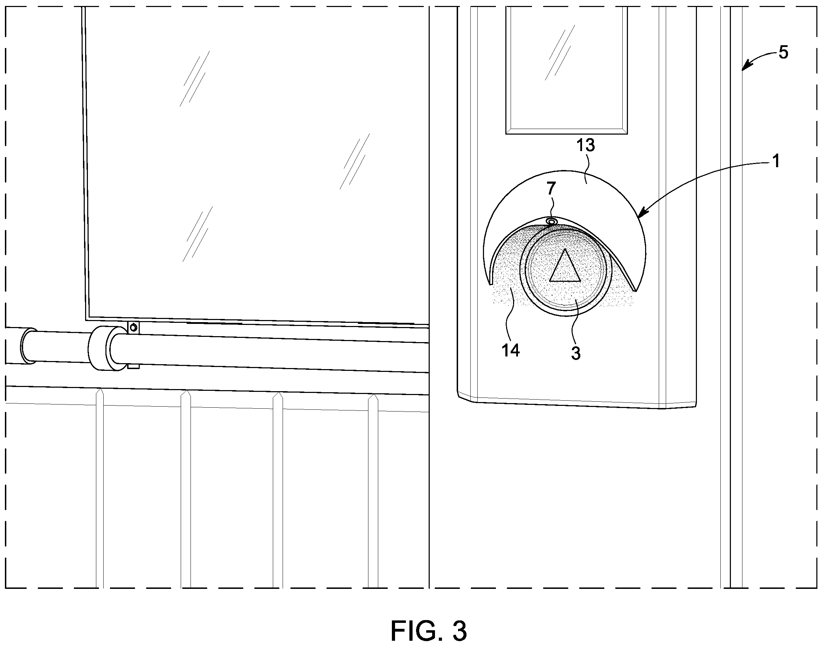

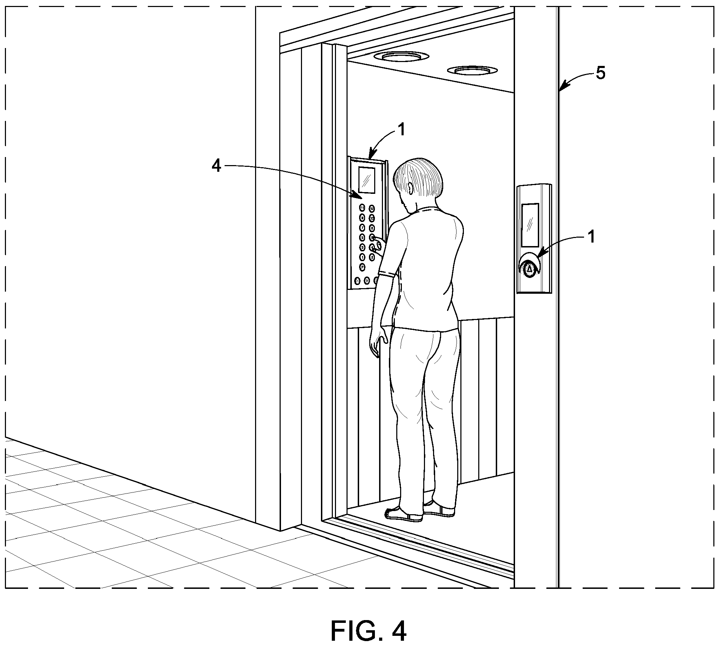

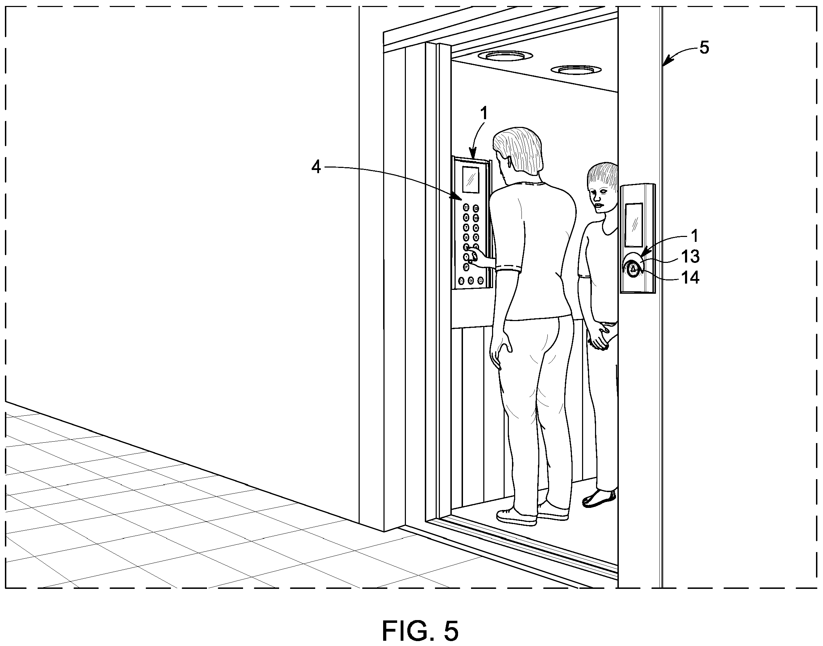

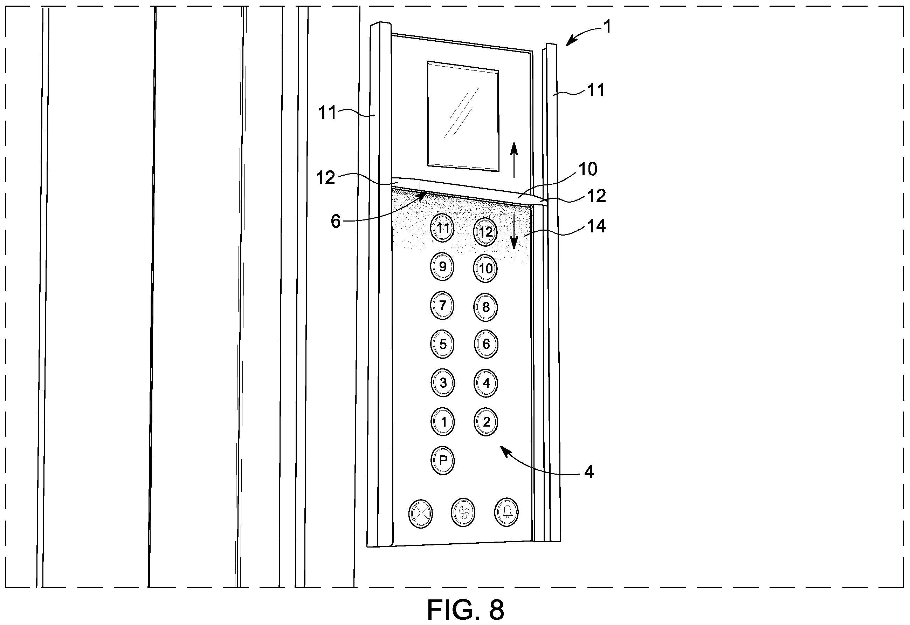

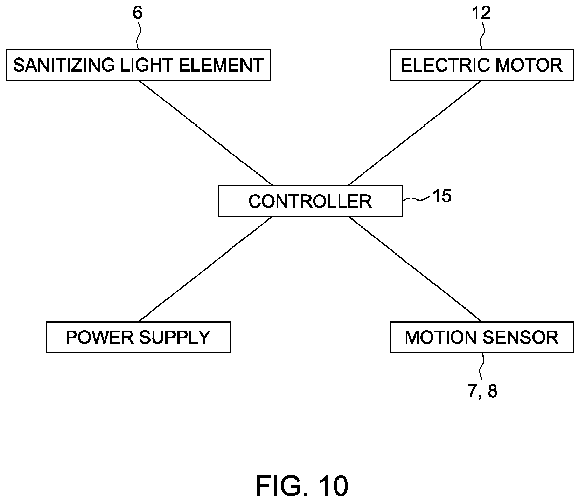

The invention will be more clearly understood from the following description of some embodiments thereof, given by way of example only, with reference to the accompanying drawings, in which Figs. 1 to 10 are views of the present invention provided as a sterilisation system for use in sterilising user interface surfaces routinely touched when users operate and use an elevator, and in particular, the user interfaces provided by a standard elevator system, including the exterior call button and the interior control panel buttons provided within the elevator enclosure, such as floor selection buttons, door control and ancillary function buttons, such as intercom, emergency call buttons and the like.
Fig. 1 is a view of an elevator system incorporating the sterilisation system according to the invention, in which a user pressing a call button for the elevator,
Fig. 2 is a view of the user entering the elevator shown in Fig. 1,
Fig. 3 is a close-up view of the call button shown in Fig. 1,
Figs. 4 and 5 are views of users selecting their desired floor level by touching a floor level button on the interior user interface control panel of the elevator,
Figs. 6 to 9 are close-up views of the interior user interface control panel of the elevator configured with the sterilisation system according to the invention in operation to sterilise the surfaces of the user interface, and
Fig. 10 is a block schematic showing electrical components of the sterilisation system according to the invention.

Referring to the drawings, there is shown a sterilisation system, indicated generally by the reference numeral 1, for sterilising a surface, indicated generally by the reference numeral 2. In the instance shown the surface 2 is provided as user interfaces of an elevator system 5 comprising an exterior call button 3 and an interior control panel 4 providing buttons such as floor selection buttons, door control and ancillary function buttons, such as intercom, emergency call buttons and the like.

It will however be understood that the present invention may be adapted for use in sterilising surfaces of keypads, access control panels for exit and entry to buildings, user terminal for operating a credit/debit card transaction, ATM interfaces and the like. Reference to the present invention being implemented and used for sterilising surfaces of user interfaces of elevator systems should in no way be seen as limiting on the scope of the invention.

The sterilisation system 1 comprises ultraviolet (UV) light emitting means 6 operable to emit UV-C light 14. As shown, a UV-light emitting means 6 is provided on the exterior-call button 3 and the interior button panel 4 so that these elevator surfaces may be sterilised.

The ultraviolet (UV) light emitting means 6 comprises one or more LED lights configured to emit UV-C light radiation 14. The LED lights may be provided as a strip of LED lights or bulbs 6 configured to emit UV-C light 14 at approximate wavelengths of between 240nm to 270nm. A wavelength of 254nm has been found to be particularly optimal in connection with the present invention, although ranges in and about this optimal wavelength are also useable for sterilising surfaces.

Also provided are sensor means 7, 8 operable to detect placement and removal of a human user's hand on the surface of the exterior call button 3 and the interior button panel 4 respectively and the UV-C light emitting means is activated on detection of placement and removal of a human user's hand on the surface of the exterior call button 3 and/or the interior button panel 4.

As shown in Figs. 1 to 3, a user is calling the elevator by actuating the call button 3. The exterior sterilization UV light emitting means 6 of the secondary exterior sterilisation unit is activated once the motion sensor 7 detects movement. Once the cycle has initiated the sensor 7 will wait approximately a predetermined period of time, such as 5, 10 or more seconds and with no movement detected will activate the LED lights of the UV light emitting means 6 for a timed period to sterilise the button 3 after the user hand is detected as having been removed.

If the motion sensor 7 detects motion while in mid cycle it will cancel the cycle, deactivate UV light emitting means 6 and the cycle will be reset to begin again after the timed period of no motion detected. A light shield or cover 13 is provided to protect the user from UV-C light waves and also to house the motion sensor 7, the controller 15 and UV emitting LED lights 6 that are directed downwardly so the UV-C light 14 covers the full area of the exterior call button 3.

With reference to the interior button panel 4 of the elevator system 5 there is provided a mounting 9 for the ultraviolet (UV) light emitting means 6, the mounting 9 comprising a mounting bar 10 and guide means 11, and the bar 10 moves relative to the guide means 11 to direct UV light 14 onto the surface 2.

The guide means 11 is provided as a pair of spaced apart slide rails 11 provided at or adjacent edges of the surface 2, and the mounting bar 10 moves along the slide rails 11 to pass over the surface 2. Moving means comprising one or more drive motors 12 operable to move the mounting bar 10 along the slide rails 11 are provided. The motors 12 may be DC motors, in which a motor is provided on each guide rail 11. The guide rails 11 may be secured to the button panel 4 by bolts, screws or other suitable attachment or securing device generally known in the art.

The mounting bar 10 may move up and down the slide rails 11, or from side to side along the slide rails 11 depending on the orientation of the rails 11 to the surface 2 being sterilised. In this way after the user has finished selecting the elevator floor, pressing the exterior call button 3, or keypad number the sterilisation system 1 is activated to sanitise the interior button panel 4 for subsequent users.

Also provided are control means 15 in the form of a processor operable to activate the moving means 12 and the ultraviolet (UV) light emitting means 6 in response to signals from the sensor means 8. When the sensor means 8 housed in the mounting bar 10 detects that the surface 2 on the button panel 4 has been touched the control means 15 is operable to activate the moving means 12 to move the mounting bar 10 up and down the slide rails 11 to simultaneously and automatically activate the UV light emitting means 6 such that UV-C light radiation 14 emitted from the UV light emitting means 6 is directed onto the surface 2 to sterilise the surface 2.

The control means 15 activates the moving means 12 and the UV light emitting means 6 in timed activation cycles as desired or required to ensure that the surfaces 2 are thoroughly sterilised.

It will be understood that the mounting bar 10 and guide means 11 are configured to move the UV light emitting means 6 over the surface 2 such that the UV light 14 is directed onto the entire area thereof to thereby sterilise the full exposed area of the surface 2 by the action of UV-C light 14 emitted by the UV light emitting means 6.

The motion sensors 8 is further configured to detect proximity of a user's hand to the surface 2 and the ultraviolet (UV) light emitting means cycle will deactivate when a user's hand is detected as being within a predetermined distance of the surface 2. In this instance the mounting bar 10 will then return to a starting position until such time as there is no further movement detected by the sensor means 8 and will restart the sterilisation cycle again to sanitise the surface 2.

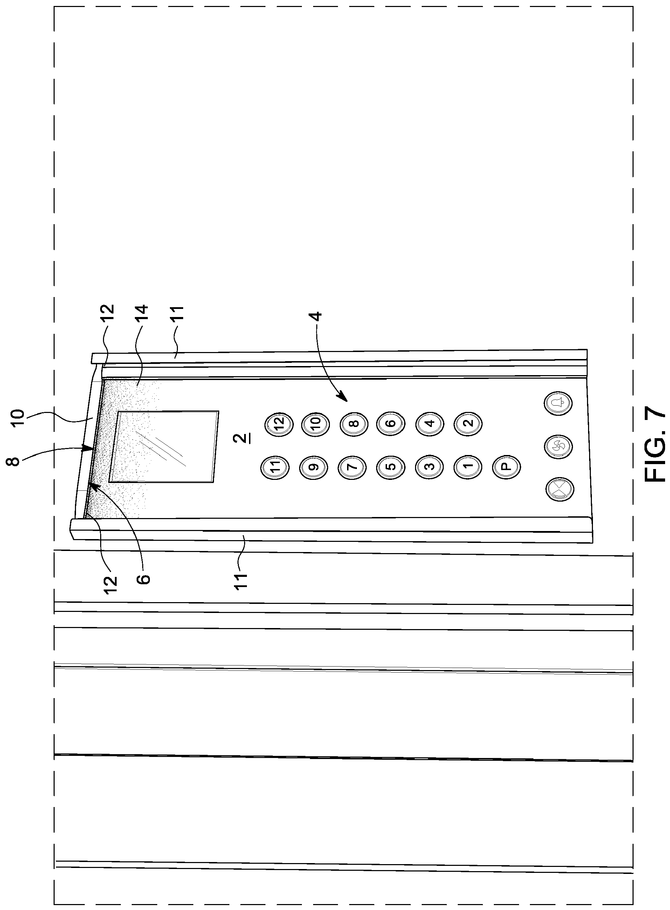

With specific reference to Figs. 7 to 9, in the instance shown, when a user engages by touching or is within proximity to the button panel surface 4, the sensor 8 detects this and signals the moving means 12 which is activated and moves the mounting bar 10 down the button panel 4. Simultaneously and automatically the UV light emitting means is activated such that UVC light radiation 14 emitted from the UV light emitting means 6 is directed onto the surface 2 to sterilise the button panel 4.

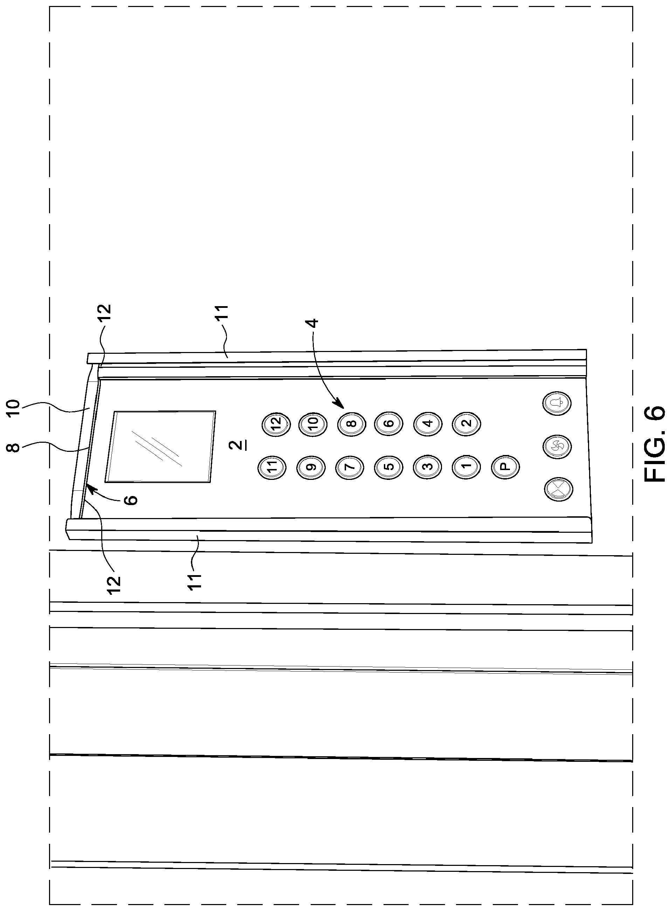

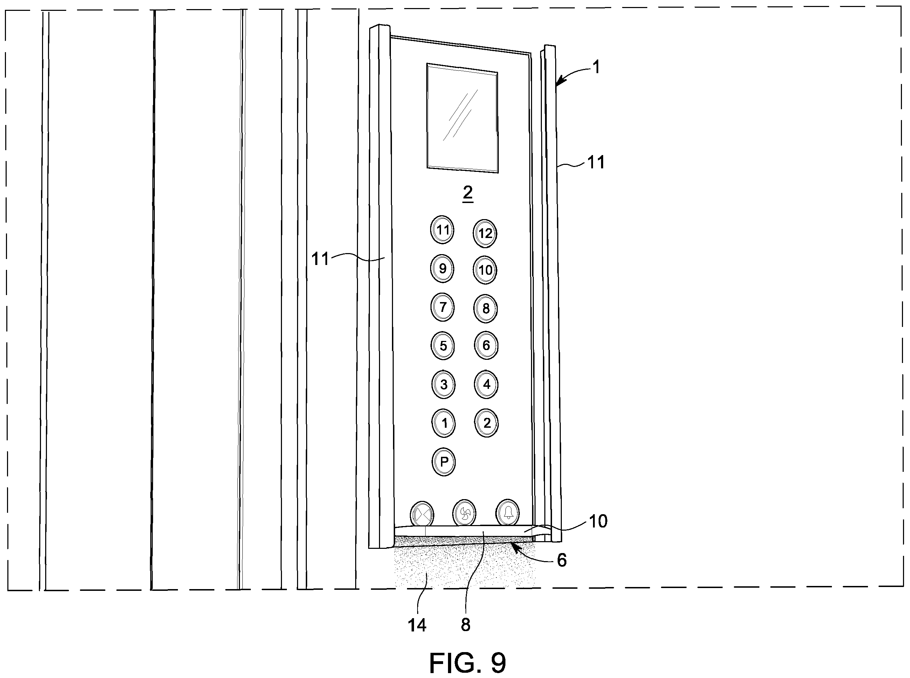

The entire exposed surface of the button panel 4 is therefore sterilised as the mounting bar traverses down the rails from the top of the guide rails (Fig. 7) to the bottom of the guide rails (Fig. 9). When completed, the mounting bar returns to the top of the guide rails 11 as shown in Fig. 6.

Aspects of the present invention have been described by way of example only and it should be appreciated that additions and/or modifications may be made thereto without departing from the scope thereof as defined in the appended claims.

## Claims

1. A sterilisation system for sterilising a surface comprising:
ultraviolet (UV) light emitting means operable to emit UV-C light radiation,
a mounting for the UV light emitting means,
moving means operable to move the mounting,
sensor means operable to detect when the surface is touched by a human,
control means operable to activate the moving means and the UV light emitting means in response to signals from the sensor means,
whereby when the sensor means detects that the surface has been touched the control means is operable to activate the moving means to move the mounting and simultaneously and automatically activate the UV light emitting means such that UV-C light radiation emitted from the UV light emitting means is directed onto the surface to sterilise the surface.

2. A sterilisation system as claimed in Claim 1, in which the control means activates the moving means and the UV light emitting means in timed activation cycles.

3. A sterilisation system as claimed in Claim 1 or Claim 2, in which the mounting comprises a mounting bar and guide means, and the bar moves relative to the guide means to direct UV light onto the surface.

4. A sterilisation system as claimed in Claim 3, in which the guide means is provided as a pair of spaced apart slide rails provided at or adjacent edges of the surface, and the mounting bar moves along the slide rails over the surface.

5. A sterilisation system as claimed in Claim 4, in which the moving means comprises one or more motors operable to move the mounting bar along the slide rails.

6. A sterilisation system as claimed in any one of the preceding claims, in which the ultraviolet (UV) light emitting means is provided on the mounting comprises one or more LED lights configured to emit UV-C light radiation.

7. A sterilisation system as claimed in any one of the preceding claims, in which the ultraviolet (UV) light emitting means is configured to emit UV-C light at approximate wavelengths of between 240nm to 270nm.

8. A sterilisation system as claimed in any one of the preceding claims, in which the ultraviolet (UV) light emitting means is configured to emit UV-C light at approximate wavelengths of 253.7nm.

9. A sterilisation system as claimed in any one of the preceding claims, in which the motion sensor is further configured to detect proximity of a user's hand to the surface and the ultraviolet (UV) light emitting means cycle will deactivate when a user's hand is detected as being within a predetermined distance of the surface, and whereby the sterilisation cycle will be deactivated and restarted when a user's hand is detected as being a predetermined distance from the sensor means.

10. A sterilisation system for sterilising user interface surfaces of an elevator system comprising:
ultraviolet (UV) light emitting means operable to emit UV-C light radiation,
a mounting for the ultraviolet (UV) light emitting means provided adjacent an interior button panel of the elevator,
moving means operable to move the mounting,
sensor means operable to detect when the surface is touched by a human,
control means operable to activate the moving means and the ultraviolet (UV) light emitting means in response to signals from the sensor means,
whereby when the sensor means detects that the surface has been touched the control means is operable to activate the moving means to move the mounting and simultaneously and automatically activate the UV light emitting means such that UV-C light radiation emitted from the UV light emitting means is directed onto the surface to sterilise the surface of the interior button panel.

11. A sterilisation system for an elevator as claimed in Claim 10, further comprising a secondary sterilisation unit comprising a further UV light emitting means and sensor means, which the secondary sterilisation unit is provided adjacent the exterior call button for the elevator.

12. A sterilisation system for an elevator as claimed in Claim 10, in which a light shield is provided with the secondary sterilisation unit to prevent a user being exposed to the emitted UV-C light radiation.
